# EUROPEAN PATENT SPECIFICATION

(11) **EP 2 260 307 B1**
(45) Date of publication and mention of the grant of the patent: **26.03.2014**
(21) Application number: 09727643.0
(22) Date of filing: 03.04.2009
(51) Int. Cl.: G01N 33/74, G01N 33/68

(54) **PRO-ENDOTHELIN-1 LEVELS FOR THE PREDICTION OF RISK OF TACHYARRHYTMIC EVENTS**
PRO-ENDOTHELIN-1-SPIEGEL FÜR DIE RISIKOBESTIMMUNG VON TACHYARRHYTHMISCHEN EREIGNISSEN
NIVEAUX PRO-ENDOTHÉLINE 1 POUR LA PRÉDICTION D'UN RISQUE D'ÉVÉNEMENTS TACHYARRHYTMIQUES

(30) Priority: 04.04.2008 EP 08154109
(43) Date of publication of application: 15.12.2010
(73) Proprietor: B.R.A.H.M.S GmbH, 16761 Hennigsdorf (DE)
(72) Inventor: STRUCK, Joachim, 13465 Berlin (DE); MORGENTHALER, Nils, 13503 Berlin (DE); BERGMANN, Andreas, 12351 Berlin (DE); MÜLLER, Christian, CH-4031 Basel (CH)
(74) Representative: Kilger, Ute
(86) International application number: PCT/EP2009/053995
(87) International publication number: WO 2009/121951

(56) References cited:
- US-A1- 2005 177 196
- SZUCS ANDREA ET AL: "Effect of incessant ventricular tachyarrhythmias on serum endothelia and big-endothelia levels" JOURNAL OF CARDIOVASCULAR PHARMACOLOGY, NEW YORK, NY, vol. 44, no. Suppl. 1, 1 November 2004 (2004-11-01), pages S402-S406, XP009100858 ISSN: 0160-2446
- ALEXIOU KONSTANTIN ET AL: "Arrhythmogenic effects induced by coronary conversion of pulmonary big endothelin to endothelin: Aggravation of this phenomenon in heritable hyperlipidemia" JOURNAL OF THE AMERICAN COLLEGE OF CARDIOLOGY, vol. 32, no. 6, 15 November 1998 (1998-11-15), pages 1773-1778, XP002484164 ISSN: 0735-1097
- BERGER RUDOLF ET AL: "B-type natriuretic peptide predicts sudden death in patients with chronic heart failure" CIRCULATION, vol. 105, no. 20, 21 May 2002 (2002-05-21), pages 2392-2397, XP002484165 ISSN: 0009-7322
- KHAN ET AL: "C-terminal pro-endothelin-1 offers additional prognostic information in patients after acute myocardial infarction" AMERICAN HEART JOURNAL, MOSBY- YEAR BOOK INC., ST. LOUIS, MO, US, vol. 154, no. 4, 1 October 2007 (2007-10-01), pages 736-742, XP022293017 ISSN: 0002-8703

## Description

The invention relates to *in vitro* methods for risk stratification for indication of device implantation, and/or antiarrhythmic hybrid therapy for a patient having a cardiac disease such as heart failure. The invention further relates to the use of such a method.

Implantable cardioverter defibrillators (ICD) effectively terminate malignant ventricular arrhythmias averting sudden death. However, ICD implantation is expensive, invasive, and associated with adverse effects such as inappropriate shocks, psychosocial problems, lead problems, and infection (Ezekowitz et al, Ann Intern Med 2007;147:251-62; Rosenqvist et al, Circulation 1998;98:663-70; Sanders GD et al, N Engl J Med 2005;353:1471-80). Of interest, the cost-effectiveness of prophylactic implantation of an ICD has even been questioned (Goldman et al, N Engl J Med 2005;353:1513-5), since up to two thirds of patients die without receiving prior ICD therapy (Ezekowitz et al, Ann Intern Med 2007;147:251-62). Consistently, risk stratification for proper indication of device implantation, and/or antiarrhythmic hybrid therapy is mandatory ( Ezekowitz et al, Ann Intern Med 2007;147:251-62).

Activation of the neurohumoral system has been linked to increased risk for malignant tachy-arrhythmias (fast ventricular tachycardia or ventricular fibrillation) in patients with systolic left ventricular (LV) dysfunction. Indeed, increased levels of B-type natriuretic peptide (BNP) indicate increased risk of sudden cardiac death (Berger et al, Circulation 2002;105:2392-7; Brunner-La Rocca et al, Eur Heart J 2001;22:1136-43). However, BNP is a quantitative marker of the severity of heart failure and indicates increased risk of death (Dao et al, J Am Coll Cardiol 2001;37:379-85), while it is not useful to correctly predict tachyarrhythmic events requiring ICD therapy before.

Elevated serum endothelin and big-endothelin levels have been observed in patients suffering or having suffered from ventricular tachyarrhythmia (Szucs et al., J Cardiovasc Pharmacol 2004, 44:S402-6).

Based on short-term experiments it has furthermore been speculated that ET and big-ET might act as mediators for arrhythmogenesis in rabbits (Alexiou et al., JACC 1998, 32:1773-8).

US 2005/0177196 A1 speculates about the use of biomarkers for assessing the risk of patients to acquire ventricular tachyrrhythmia. However, this application does not provide specific biomarkers in this context.

Thus, it was an object of the present invention to find a marker predicting tachyarrhythmic events in patients having a cardiac disease as e.g. chronic heart failure.

Surprisingly, it was found that increased levels of CT-proET-1 may predict the occurrence of a first malignant ventricular tachyarrhythmic event requiring device therapy. Increased levels of CT-proET-1 independently predict the future occurrence of malignant tachyarrhythmias in ICD patients with heart failure and reduced LV-EF. Further, measurement of CT-proET-1 levels may be very useful for risk stratification of patients requiring an ICD for primary prevention of sudden arrhythmic death.

Thus, subject of the present invention is an *in vitro* method for risk stratification for indication of ICD device implantation, and/or antiarrhythmic hybrid therapy for a patient having a cardiac disease comprising:
- determining the level of CT-proET-1 or fragments thereof of at least 12 amino acids in a sample obtained from a patient.

In case the level of fragments of CT-proET-1 are determined these fragments comprise at least 12 amino acids, preferably at least 20 amino acids, were preferably at least 30 amino acids. Endothelin (ET)-1 is a potent endothelium-derived endogenous vasoconstrictor (Yanagisawa M, Kurihara H, Kimura S, Goto K, Masaki T. A novel peptide vasoconstrictor, endothelin is produced by vascular endothelium and modulates smooth muscle Ca2+ channels. J Hypertens Suppl 1988;6:S188-91.). ET-1 exerts its vascular effects by activation of ET(A) and ET(B) receptors on smooth muscle cells, which causes an increase in intracellular calcium (Yanagisawa et al, J Hypertens Suppl 1988;6;5188-91). Mature Endothelin-1 is derived from a larger precursor termed Pro-Endothelin-1. Pro-Endothelin-1 can be proteolytically processed into various fragments as described (Struck J, Morgenthaler NG, Bergmann A. Proteolytic processing pattern of the endothelin-1 precursor in vivo. Peptides. 2005 Dec;26(12):2482-6.). These fragments are subject to proteolytic degradation in the blood circulation, which can happen quickly or slowly, depending on the type of fragment and the type and concentration/activity of proteases present in the circulation. One example of these fragments is C-terminal pro-Endothelin-1 (CT-proET-1), which can be measured by a sandwich immunoassay (Papassotiriou J, Morgenthaler NG, Struck J, Alonso C, Bergmann A. Immunoluminometric assay for measurement of the C-terminal endothelin-1 precursor fragment in human plasma, Clin Chem. 2006 Jun;52(6):1144-5 1.)

Continuous variables are expressed as mean±standard deviation (SD) or expressed as median (range) and point estimate (95% confidence intervals) as indicated. The objective was to examine whether CT-proET-1 contributes to predict time to first malignant tachyarrhythmic event. Malignant tachyarrhythmias were defined as fast ventricular tachyarrhythmias (VT or VF) with cycle lengths ≤250ms ms (heart rate ≥240 bpm) probably leading to death if not terminated by the device (Bocker D, Block M, Isbruch F, et al. Do patients with an implantable defibrillator live longer? J Am Coll Cardiol 1993,21:1638-44.). Univariate and multivariable Cox proportional hazards models were used to evaluate the associations between the outcome measures.

In one embodiment of the method according to the present invention said level of CT-proET-1 or fragments thereof of at least 12 amino acids is correlated to the risk of first malignant tachyarrhythmias.

The objective of said method is the stratification of patients according to their requirement for an implantable cardioverter defibrillator (ICD), and/or an antiarrthmic hybrid therapy.

According to a preferred embodiment of the invention said correlating comprises comparing said level of CT-proET-1 or fragments thereof of at least 12 amino acids to a threshold level, whereby, when said level of CT-proET-1 or fragments thereof of at least 12 amino acids exceeds said threshold level, said patient is at high risk of tachyarrhythmia.

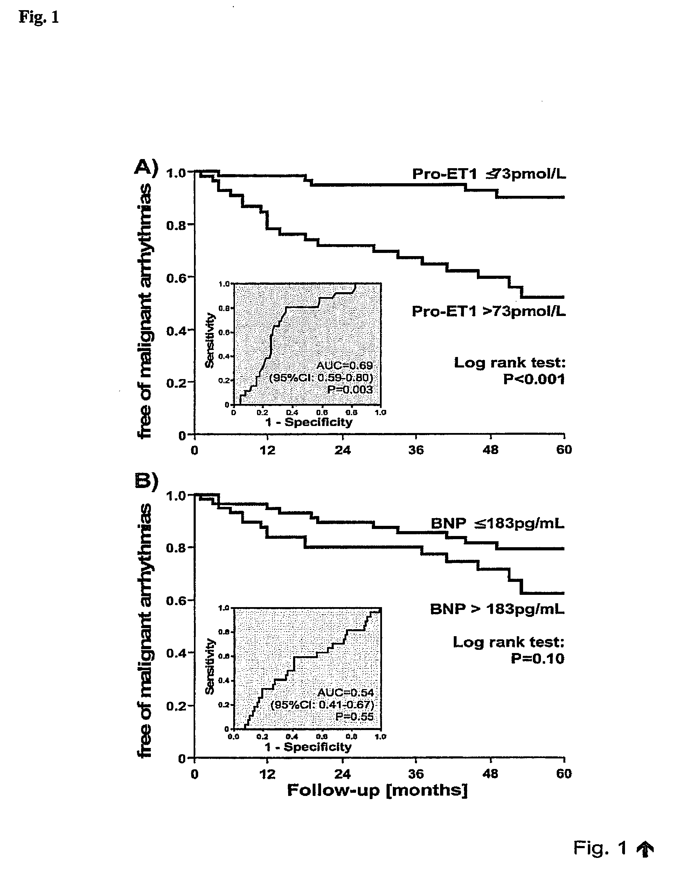

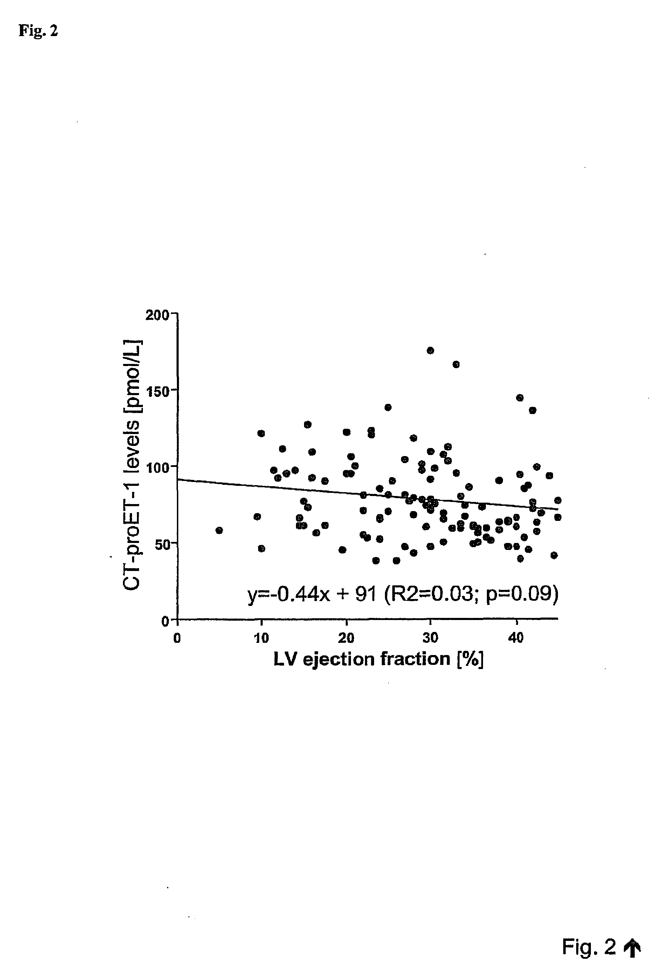

ICD patients have been stratified into two groups according to CT-proET-1 or BNP levels and Kaplan Meier analysis has been performed. Risk of tachyarrhythmia was significantly higher in patients with CT-proET-1 plasma levels >73pmol/L (median) than in patients with CT-proET-1 values below this cut point (logrank test, p<0.001, Fig. 1A). No significant differences were found in patients with BNP plasma levels ≤183 pg/mL (median) or >183pg/mL using malignant tachyarrhythmia as endpoint (logrank test, p=0.10, Fig. 1B).. No significant associations of LV ejection fraction and CT-proET-1 levels were found (Fig. 2). The area under the ROC curve that used CT-proET-1 levels to predict the occurrence of a first malignant tachyarrhythmia was 0.69 (Fig. 1A, insert). The optimum concentration or CT-proET-1 for the calculation of positive and negative predictive accuracy as obtained from the ROC curve was 75.5 pmol/L (sensitivity 80.8%, specificity 64.4%, negative predictive value 92.1%, positive predictive value 39.6%, and a positive likelihood ration of 2.27). CT-proET-1 plasma levels significantly contributed to multivariable Cox regression models predicting the risk of first malignant tachyarrhythmia. Risk ratio has been calculated per pmol/L increment of CT-proET-1 levels (Table 2). Using log CT-proET-1 in statistical analysis did not change obtained results. Multivariable Cox regression analysis revealed that treatment with betablockers indicates favourable prognosis (Table 2).

Thus, in a preferred embodiment of the invention said threshold level is at about 73 +/- 20% pmol/L.

According to a preferred embodiment the cardiac disease is any condition, for which the implantation of an ICD is potentially indicated, inlcuding heart failure of either ischemic or non-ischemic etiology. According to a preferred embodiment the patients have a LV ejection fraction ≤45%.

The sample obtained from a patient is preferably a sample selected from the group comprising a blood sample, a serum sample, and a plasma sample.

Further, a subject of the present invention is a method further comprising combining said level of CT-proET-1 or fragments there of at least 12 amino acids with the level/condition of one or more additional prognostic markers, parameters or factors, whereby the combination of said level of CT-proET-1 or fragments thereof of at least 12 amino acids with said level/condition of one or more additional prognostic markers, parameters or factors increases the predictive value for risk of said method.

In a preferred embodiment, the additional prognostic marker, parameter or factor is selected from a group comprising all parameters listed in Table 2, which exhibit a p<0.05 in the univariate or multivariate analysis, whereby BNP is only an example for proBNP or fragments thereof of at least 12 amino acids including BNP or NT-proBNP.

Preferably, said level of CT-proET-1 or fragments thereof of at least 12 amino acids is measured with a sandwich immunoassay. Preferably, C-terminal ET-1 precursor fragment (CT-proET-1), which indirectly estimates activity of the endothelin system, is measured. In a preferred embodiment 2 antibodies are used in this assay, preferably polyclonal antibodies. In a preferred embodiment the antibodies are directed to amino acids 168-212 of pre-proET-1. This method may be conducted with a commercially available sandwich immunoassay (CT-proET-1 LIA, B.R.A.H.M.S AG, Hennigsdorf, Berlin, The assay (normal reference range: median: 44.3, range: 10.5-77.4 pmol/L) has an analytical detection limit of 0.4 pmol/L and the intra-assay CV were <10% for values >10 pmol/L (Papassotiriou J, Morgenthaler NG, Struck J, Alonso C, Bergmann A. Immmioluminometric assay for measurement of the C-terminal endothelin-1 precursor fragment in human plasma. Clin Chem 2006;52:11.44-51.).

In the context of the present invention, a "cardiac disease" is a pathological state relating to the cardiac system, e.g. the heart and/or blood vessels, of a patient Such cardiac diseases may for instance be congenital heart defects, coronary artery disease, heart failure and/or valvular heart disease.

In the present invention, the term "primary prevention" means to avoid the development of a disease in an otherwise healthy or at least non-symptomatic individual.

In the context of the present invention, a malignant tachyarrhythmia is preferably defined as fast ventricular tachyarrhythmia (VT or VF) with a cycle length ≤250ms (heart rate ≥240 bpm).

In the present invention, the term "risk stratification" denotes an assignment of a probability to experience certain adverse events to an individual. Hereby, the individual may preferably be accounted to a certain risk category, wherein categories comprise for instance high risk versus low risk, or risk categories based on numeral values, such as risk category 1, 2, 3, etc.

In the context of the present invention, terms such as "predictive value" refer to the statistic significance of a certain determined result of a measurement. Thus, an increase in predictive value or predictive power in the context of the present invention means that the probability of a correct diagnosis, prognosis, stratification or the like based on a certain value determined from the measurement of the level of a certain marker in a sample increases.

As mentioned herein, an "assay" or "diagnostic assay" can be of any type applied in the field of diagnostics, including but not restricted to assays methods based on enzymatic reactions, luminescence, fluorescence or radiochemicals. The preferred detection methods comprise strip tests, radioimmunoassays, chemiluminescence- and fluorescence- immunoassays, Immunoblot assays, Enzyme-linked immunoassays (ELISA), Luminex-based bead arrays, and protein microarray assays. The assay types can further be microtitre plate-based, chip-based, bead-based, wherein the markers can be attached to the surface or in solution. The assays can be homogenous or heterogeneous assays, sandwich assays, competitive and non-competive assays. In a particularly preferred embodiment, the assay is in the form of a sandwich assay, which is a noncompetitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody on the site is then measured by an appropriate method. The general composition and procedures involved with "sandwich assays" are well-established and known to the skilled person.
(The Immunoassay Handbook, Ed. David Wild, Elsevier LTD, Oxford; 3rd ed. (May 2005), ISBN-13: 978-0080445267; Hultschig C et al., Curr Opin Chem Biol. 2006 Feb;10(1):4-10. PMID: 16376134), incorporated herein by reference.

In a particularly preferred embodiment the assay comprises two capture molecules, preferably antibodies which are both present as dispersions in a liquid reaction mixture, wherein a first marking component is attached to the first capture molecule, wherein said first marking component is part of a marking system based on fluorescence- or chemiluminescence-quenching or amplification, and a second marking component of said marking system is attached to the second capture molecule, so that upon binding of both capture molecules to said protease or said endogenous inhibitor or said complex of the protease with an endogenous inhibitor, a measurable signal is generated that allows for the detection of the formed sandwich complexes in the solution comprising the sample.

Even more preferred, said marking system comprises rare earth cryptates or rare earth chelates in combination with a fluorescence dye or chemiluminescance dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3,5, CY5.5, Cy7, Xanthen, 6-Carboxy-2',4',7',4,7-hexachlorofluorescein (HEX), TET, 6-Carboxy-4',5'-dichloro-2',7'-di-methadyfluorescein (JOE), NN,N',N'-Tetramethyl-6-carboxyrhodamine (TAMRA), 6-Carboxy-X-rhodamme (ROX), 5-Carboxyrhodamine-6G (R6G5), 6-carboxyrhodamine-6G (RG6), Rhodamine,Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles are described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, including citations on pages 551-562.

As used herein, the term "hybrid therapy" refers to a combination therapy, wherein pharmaceutical therapy and device therapy are combined in one patient.

"Patients" in the meaning of the invention are understood to be all persons or animalsirrespective whether or not they exhibit pathological changes, unless stated otherwise. In the meaning of the invention, any sample collected from cells, tissues, organs, organisms or the like can be a sample of a patient to be diagnosed. In a preferred embodiment the patient according to the invention is a human.

As mentioned herein in the context of proteins or peptides, the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds.

In the context of the present invention, the term "level" in expressions such as "level of a protease", "analyte level" and similar expressions, refers to the quantity of the molecular entity mentioned in the respective context.

As used herein, terms such as "marker" "prognostic marker(s), parameter(s) or factor(s)" or "biomarker" or "biological marker" are used interchangeably and relate to measurable and quantifiable biological parameters (e.g., specific enzyme concentration, specific hormone concentration, specific gene phenotype distribution in a population, presence of biological substances) which serve as indices for health- and physiology-related assessments, such as disease risk, psychiatric disorders, environmental exposure and its effects, disease diagnosis, metabolic processes, substance abuse, pregnancy, cell line development, epidemiologic studies, etc. Furthermore, a biomarker is defined as a characteristic that is objectively measured and evaluated as an indicator of normal biological processes, pathogenic processes, or pharmacologic responses to a therapeutic intervention. A biomarker may be measured on a biosample (as a blood, urine, or tissue test), it may be a recording obtained from a person (blood pressure, ECG, or Holter), or it may be an imaging test (echocardiogram or CT scan) (Vasan et al. 2006, Circulation 113:2335-2362).

Biomarkers can indicate a variety of health or disease characteristics, including the level or type of exposure to an environmental factor, genetic susceptibility, genetic responses to exposures, biomarkers of subclinical or clinical disease, or indicators of response to therapy. Thus, a simplistic way to think of biomarkers is as indicators of disease trait (risk factor or risk biomarker), disease state (preclinical or clinical), or disease rate (progression). Accordingly, biomarkers can be classified as antecedent biomarkers (identifying the risk of developing an illness), screening biomarkers (screening for subclinical disease), diagnostic biomarkers (recognizing overt disease), staging biomarkers (categorizing disease severity), or prognostic biomarkers (predicting future disease course, including recurrence and response to therapy, and monitoring efficacy of therapy). Biomarkers may also serve as surrogate end points. A surrogate end point is one that can be used as an outcome in clinical trials to evaluate safety and effectiveness of therapies in lieu of measurement of the true outcome of interest. The underlying principle is that alterations in the surrogate end point track closely with changes in the outcome of interest. Surrogate end points have the advantage that they may be gathered in a shorter time frame and with less expense than end points such as morbidity and mortality, which require large clinical trials for evaluation. Additional values of surrogate end points include the fact that they are closer to the exposure/intervention of interest and may be easier to relate causally than more distant clinical events. An important disadvantage of surrogate end points is that if clinical outcome of interest is influenced by numerous factors (in addition to the surrogate end point), residual confounding may reduce the validity of the surrogate end point. It has been suggested that the validity of a surrogate end point is greater if it can explain at least 50% of the effect of an exposure or intervention on the outcome of interest. For instance, a biomarker may be a protein, peptide or a nucleic acid molecule.

### Figure Description

### Figure 1

Heart failure patients have been stratified into two groups according to CT-proET-1 and BNP levels and Kaplan Meier analyses have been performed. A) Patients with CT-proET-1 plasma levels >73pmol/L (median) had a significantly worse outcome than patients with CT-proET-1 values below this cut point (logrank test) regarding malignant tachyarrhythmic events as endpoint. B) Patients with BNP plasma levels ≥ 183 pg/mL (median) did not have a higher rate of malignant ventricular tachyarrhythmic events than patients below this cut point (logrank test). Respective ROC curve analyses are shown in the inserts.

### Figure 2

Linear regression analysis of LV ejection fraction and CT-proET-1 levels in 123 heart failure patients with implantable cardioverter defibrillators.

### Examples

### Methods

### Patients, study design

The study population consisted of 123 defibrillator patients with heart failure of ischemic or nonischemic etiology that were prospectively recruited at the defibrillator outpatient clinic of the University of Marburg between June 2002 and September 2003 (Christ et al, Eur J Heart Fail 2007;9:272-9). The study was conducted according the principles of Good Clinical Practice and the Declaration of Helsinki, and patients gave their informed consent before enrolment into the study (Christ et aI, Eur J Heart Fail 2007;9:272-9).

### Electrocardiography and echocardiography

Standardised 12-lead electrocardiograms (ECGs) were recorded at a paper speed of 50 mm/second from each patient and interpreted by a single experienced researcher blinded to outcome. Patients were classified to have pacemaker rhythm, if more than 50% of beats were paced during ECG recording. Two-dimensional echocardiographic images of the heart were obtained at the end of the expiratory period using a Vingmed Vivid Five machine (GE Medical Systems, Solingen, Germany) and parameters measured using standard procedures (Christ et al, Eur J Heart Fail 2007;9:272-9).

### Laboratory Procedures

After overnight fasting, venous blood samples were drawn between 8:30h and 10:30h through a 21-gauge cannula inserted into an antecubital vein using ethylenediamine tetraacetic acid (EDTA) containing monovettes (Sarstedt, Nuembrecht, Germany). Patients lay for at least 15 min in a supine body position. EDTA blood samples were immediately transferred on chilled ice, plasma separated using a centrifuge and plasma stored at -80 °C until analysis.

BNP levels were determined using a chemiluminescence immunoassay (Bayer AG, Fernwald, Germany). The analytical detection limit of the assay was 2 pg/mL. The intra-assay coefficient of variations (CVs) were 1.8-4.3% at 29.4-1763 pg/mL and the interassay CVs were 2.3-4.7% at 29.4-1736 pg/mL BNP (Christ et al, Eur J Heart Fail 2007;9:272-9). Endothelin-1 is difficult to measure due to instability and receptor binding. C-terminal ET-1 precursor fragment (CT-proET-1), which indirectly estimates activity of the endothelin system, was measured with a novel sandwich immunoassay (CT-proET-1 LIA, B.R.A.H.M.S AG, Hennigsdorf, Berlin, Germany) (Papassotiriou et al, Clin Chem 2006;52:1144-51)) using 2 polyclonal antibodies directed to amino acids 168-212 of pre-proET-1. The assay (normal reference range: median: 44.3, range: 10.5-77.4pmol/L) has an analytical detection limit of 0.4pmol/L and the intra-assay CV were <10% for values >10pmol/L (Papassotiriou et al, Clin Chem 2006;52:1144-51).

### Defibrillator implantation and device programming

Exclusively, ICDs with non-thoracotomy lead systems and biphasic shock waveforms with maximum shock energy of 27 to 34 J were used. All ICD systems used provided stored intracardiac electrograms in addition to beat-to-beat intervals of episodes triggering device action (Guidant, St. Paul, Minnesota; Medtronic, Minneapolis, Minnesota). The time interval for VF detection ranged from one to three seconds in Guidant devices; the Medtronic devices were programmed to require 18 out of 24 beats below the programmed detection cycle length (range 240 - 280ms) in order to initiate capacitor charging in the VF zone. Slower ventricular tachyarrhythmias were detected and treated in one or two separate VT zones with anti-tachycardia burst pacing followed by up to 4 cardioversion shocks (Grimm et al, J Am Coll Cardiol 2002;39:780-7).

### Follow-up

All data of baseline clinical characteristics, including the results of cardiac evaluation and implantation data had been collected prospectively in the Marburg Defibrillator Database (Christ et al, Eur J Heart Fail 2007;9:272-9, Grimm et al, J Am Coll Cardiol 2002;39:780-7). Follow-up in this study started at the time of biomarker measurement, was up to 60 months and could be completed in all patients enrolled. Patients were followed primarily in our defibrillator outpatient clinic in three- to six months intervals or as soon as possible after spontaneous ICD shocks for device interrogation and retrieval of stored electrocardiograms. Two experienced electrophysiologists classified all stored electrocardiograms of episodes triggering ICD therapy or inappropriate using previously described criteria (Christ et al, Eur J Heart Fail 2007;9:272-9; Marchlinski et al, Pacing Clin Electrophysiol 1993;16:527-34).

### Statistical Analysis

Continuous variables are expressed as mean±standard deviation (SD) or expressed as median (range) and point estimate (95% confidence intervals) as indicated. The primary objective was to examine whether biomarker levels including CT-proET-1 or BNP contribute to predict time to first malignant tachyarrhythmic event. Malignant tachyarrhythmias were defined as fast ventricular tachyarrhythmias (VT or VF) with cycle lengths ≤250ms (heart rate ≥240 bpm) probably leading to death if not terminated by the device (Bocker et al, J Am Coll Cardiol 1993;21:1638-44.). Univariate and multivariable Cox proportional hazards models were used to evaluate the associations between the outcome measures.

For building a first Cox regression model, we thereby used a stepwise procedure with an entry level of 0.05. The p value for staying in the model was set at 0.05. Event probabilities were estimated with the Kaplan-Meier method and the log-rank test was used for comparisons of curves. Additional statistical analyses were used for exploration, description, and interpretation. Comparisons between groups were done by the Students t-test, U-test or chi-square test as applicable. All p-values reported are two-sided, a p-value <0.05 was considered significant.

Receiver operator characteristic (ROC) curves were constructed to assess the sensitivity and specificity of biomarkers throughout the concentrations to detect respective endpoints. All statistical calculations were performed using the SPSS statistical software package (version 14.0; SPSS Inc., Chicago, IL).

### Results

Demographic characteristics of the overall study cohort are shown in table 1. During a maximum follow-up of up to 60 months (median 51 months) after enrolment, 27 patients had first malignant tachyarrhythmic events CT-proET1 levels were significantly higher in patients with (median: 90pmol/L) compared to patients without a first malignant tachyarrhythmic event (median: 67pmol/L; p=0.003). In contrast, no differences were found between groups for BNP (p=0.55) (Table 1).

### Predictors of first malignant tachyarrhythmic events

Malignant tachyarrhythmias occurred at a median of 18 months after study enrolment (IQR: 8-41 months). CT-pro-ET-1 levels, NYHA class, increased jugular venous pressure, and treatment with betablockers significantly contributed to univariate Cox regression models to predict the primary endpoint. Neither BNP, nor systolic blood pressure, LV enddiastolic diameter, LV-EF, or the presence of a left bundle branch block (LBBB) predicted risk of malignant tachyaarrhythmias requiring ICD therapy (Table 2). ICD patients have been stratified into two groups according to CT-proET-1 or BNP levels and Kaplan Meier analysis has been performed. Risk of tachyarrhythmia was significantly higher in patients with CT-proET-1 plasma levels >73pmol/L (median) than in patients with CT-proET-1 values below this cut point (logrank test, p<0.001, Fig. 1A). No differences were found in patients with BNP plasma levels ≤83 pg/mL (median) or >183pg/mL using malignant tachyarrhythmia as endpoint (logrank test, p=0.10, Fig. 1B). No significant associations of LV ejection fraction and CT-proET-1 levels were found (Fig. 2).

The area under the ROC curve that used CT-proET-1 levels to predict the occurrence of a first malignant tachyarrhythmia was 0.69 (Fig. 1A, insert). The optimum concentration of CT-proET-1 for the calculation of positive and negative predictive accuracy as obtained from the ROC curve was 75.5pmol/L (sensitivity 80.8%, specificity 64.4%, negative predictive value 92.1 %, positive predictive value 39.6%, and a positive likelihood ration of 2.27). CT-proET-1 plasma levels significantly contributed to multivariable Cox regression models predicting the risk of first malignant tachyarrhythmia. Risk ratio has been calculated per pmol/L increment of CT-proET-1 levels (Table 2). Using log CT-proET-1 in statistical analysis did not change obtained results. Multivariable Cox regression analysis revealed that treatment with beta blockers indicates favourable prognosis (Table 2).

**Table 1**

| Clinical characteristics of patients with implantable cardioverter/defibrillator implantation at study entry. | | | | | |
|---|---|---|---|---|---|
| **Variable** | | **All patients** | **Malignant tachyarrhythmia** | | |
| | | | **Event** | **Event-free** | **P=** |
| Number | | 123 | 27 | 96 | |
| Age, years | | 63±12 | 64±13 | 63±12 | 0.66 |
| Male sex (%) | | 104 (85) | 21 (78) | 83 (87) | 0.36 |
| Weight, kg | | 82±14 | 82±13 | 82±14 | 0.83 |
| Body mass index, kg/m² | | 26.8±4.2 | 27.6±4.0 | 26.6+4.2 | 0.29 |
| Systolic blood pressure, mmHg | | 132±21 | 130±18 | 132±22 | 0.53 |
| **Dyspnea** NYHA class I/II (%) | | 75 (61) | 13 (48) | 62 (65) | 0.08 |
| | NYHA class III (%) | 45 (37) | 12 (44) | 33 (34) | |
| | NYHA class IV (%) | 3 (2) | 2 (7) | 1 (1) | |
| **Cause of HF** | non-ischemic (%) | 63 (51) | 15 (56) | 48 (53) | 0.83 |
| **Clinical chemistry** | | | | | |
| Hemoglobin, g/L | | 141±16 | 144±17 | 140±16 | 0.21 |
| Sodium, mmol/L | | 139±3 | 139±3 | 140±4 | 0.29 |
| Potassium, mmol/L | | 4.0±0.4 | 4.0±0.5 | 4.0±0.4 | 0.61 |
| Magnesium, mmol/L | | 0.8±0.1 | 0.8±0.1 | 0.8±0.1 | 0.54 |
| Creatinine clearance, mL/min | | 90±37 | 89±37 | 90±38 | 0.89 |
| **CT pro-endothelin 1**, pmol/L (median) | | 73 | 90 | 67 | 0.003 |
| | IQR | 59-95 | 76-99 | 57-92 | |
| **B-type natriuretic peptide,** pg/mL (median) | | 183 | 221 | 177 | 0.55 |
| | IQR | 77 - 395 | 75-482 | 78-357 | |

| **12-lead electrocardiogram (%)** | | | | | |
|---|---|---|---|---|---|
| Sinus rhythm | | 69 (55) | 15 (56) | 54 (56) | 1.0 |
| Atrial fibrillation | | 31 (25) | 7 (26) | 24 (25) | 1.0 |
| Pacemaker stimulation | | 37 (30) | 6 (22) | 31 (32) | 0.35 |
| Left bundle branch block | | 42(34) | 9 (33) | 33(34) | 1.0 |

| **Echocardiographic study** | | | | | |
|---|---|---|---|---|---|
| LV EDD, mm | | 66±10 | 67±9 | 65±11 | 0.45 |
| LV ejection fraction, % | | 29±10 | 28±9 | 29±10 | 0.53 |

| **Medication** | | | | | |
|---|---|---|---|---|---|
| Diuretics (%) | | 103 (83.7) | 23 (85.2) | 80 (83.3) | 1.00 |
| ACE-I, ARB (%) | | 100 (81.3) | 19 (70.4) | 81 (84.4) | 0.16 |
| Betablocker (%) | | 92 (74.8) | 15 (55.6) | 77 (80.2) | 0.01 |
| Digitalis (%) | | 76 (61.8) | 18 (66.7) | 58 (60.4) | 0.66 |
| Spironolactone (%) | | 43 (35.0) | 7 (25.9) | 36 (37.5) | 0.36 |
| Amiodarone (%) | | 29 (23.6) | 5 (18.5) | 24 (25.0) | 0.61 |
| Statins (%) | | 46 (37.4) | 7 (25.9) | 39 (40.6) | 0.18 |
| Class I antiarrhythmics (%) | | 4 (3.3) | 1 (3.7) | 3 (3.1) | 1.00 |

| | | | | | |
|---|---|---|---|---|---|
| NYHA, New York Heart Association; LV, left ventricular; CT, C-terminal, EDD, enddiastolic diameter, ACE-I, angiotensin converting enzyme inhibitor, ARB, angiotensin receptor blocker. Statistical analysis has been performed as outlined in the statistics section. Values are displayed as mean±SD or as indicated. | | | | | |

**Table 2: Tachyarrhythmic events in ICD patients during follow-up**

| | | **First malignant tachyarrhythmic event** | | |
|---|---|---|---|---|
| Parameter | | Hazard ratio Point estimate (95% CI) | P univariate | P multivariable |
| Age, years | | 1.01 (0 98-1.05) | 0.51 | |
| Male sex | | 0.58 (0 23-1 43) | 024 | - |
| Body mass index kg/m² | | 1.05 (0.95-1.16) | 032 | - |
| Medical history | Coronary artery disease/prior myocardial infarction | 0.83 (0 38-1 78) | 0.63 | - |
| | Successful resuscitation prior to ICD implantation | 0 52 (0.20-1.36) | 0.18 | - |
| | Syncope prior to ICD implantation | 1.54 (0 72 3.29) | 027 | - |
| | Presence of malignant arrhythmias before enrolment | 1.14 (0.51-2.55) | 074 | - |
| | ICD for primary prevention of malignant arrhythmias | 1 13 (0.52-2 43) | 076 | - |
| | Smoking | 0.63 (0.22-1.83) | 040 | - |
| | Hypertension | 0.57 (0.26-1.28) | 017 | - |
| | Diabetes mellitus | 1.45 (0.68-3 09) | 033 | - |
| | Increased jugular venous pressure | 392 (1.34-11.48) | 0.01 | 0.38 |
| | Systolic blood pressure, mmHg | 0.99 (0.97-1.01) | 0.50 | |
| | New York Heart Association | 216 (1.13-4.14) | 0.02 | 0.08 |
| Medication | On treatment with ACI or ARBs | 0 48 (0.21-1.09) | 0.08 | - |
| | On treatment with loop diuretics | 1 48 (0 69-3.15) | 0.31 | - |
| | On treatment with spironolactone | 0 59 (0.25-1.40) | 023 | - |
| | On treatment with betablockers | 0 29 (0.14-0.63) | 0.002 | 0.01 |
| | On treatment with amiodarone | 0.81 (0.31-2.15) | 0.68 | - |
| | On treatment with statins | 0 53 (0.22-1.25) | 0.14 | - |
| 12 lead ECG | Presence of atrial fibrillation | 1.06 (0 45-2 50) | 0.90 | - |
| | Left bundle branch block | 0 93 (0.42-2.06) | 0.85 | - |
| Echocardiography | LV end-diastolic diameter mm | 1 02 (0.98-1.05) | 032 | - |
| | LV ejection fraction % | 0 99 (0.95-1.02) | 0.42 | - |
| Clinical chemistry | Haemoglobin g/L | 1.02 (0.99-1.04) | 0.27 | - |
| | Potassium mmol/L | 0.71 (0.30 1.68) | 043 | - |
| | Magnesium, mmol/L | 0.24 (0.002 27 08) | 0.55 | - |
| | Creatinine clearance (ml/min) | 0.99 (0 98-1.01) | 0.59 | - |
| | C-terminal pro-endothelin-1, pmol/L | 1.03 (1.01-1.04) | <0.001 | 0.02 |
| | B-type natriuretic peptide pg/mL | 1.001 (1.000-1.002) | 0.19 | - |

| | | | | |
|---|---|---|---|---|
| Hazard ratios for the primary endpoint (first malignant tachyarrhythmic event) during 60 months of follow up (n=123; LV, left ventricular; ICD, unplantable cardioverter defibrillator) | | | | |

## Claims

1. An in vitro method for risk stratification for the occurrence of malignant tachyarrhythmias for a patient having a cardiac disease comprising:
• determining the level of CT-proET-1 or fragments thereof of at least 12 amino acids in a sample obtained from a patient.

2. An *in vitro* method according to claim 1, wherein said level of CT-proET-1 or fragments thereof of at least 12 amino acids is correlated to the risk of malignant tach-yarrhythmias requiring implantation of an implantable cardioverter defibrillator (ICD), and/or an antiarrhythmic hybrid therapy.

3. An *in vitro* method according to claim 2, for primary prevention of sudden arrythmic death.

4. A method according to claim 2 or 3, wherein said correlating step comprises comparing said level of CT-proET-1 or fragments thereof of at least 12 amino acids to a threshold level, whereby, when said level of CT-proET-1 or fragments thereof of at least 12 amino acids or fragments thereof exceeds said threshold level, said patient is at high risk of tachyarrhythmia

5. A method according to claim 4, wherein said threshold level is at about 73 +/-20% pmol/L.

6. A method according to claims 1 -5, wherein the cardiac disease is heart failure of either ischemic or non-ischemic etiology.

7. A method according to claims 1-6, wherein the patients have a LV ejection fraction ≤ 45%.

8. A method according to any of the preceding claims, wherein said sample is selected from the group comprising a blood sample, a serum sample, and a plasma sample.

9. A method according to any of the preceding claims, further comprising combining said level of CT-proET-1 or fragments thereof of at least 12 amino acids with the lev-el/condition of one or more additional prognostic markers, parameters or factors, whereby the combination of said level of CT-proET-1 or fragments thereof of at least 12 amino acids with said level/condition of additional prognostic marker(s), parameter(s) or factor(s) increases the predictive value for risk of said method.

10. A method according to claim 9, wherein the additional prognostic marker, parameter or factor is selected from a group comprising all parameters listed in Table 2, which exhibit a p<0.05 in the univariate or multivariate analysis, whereby BNP is only an example for proBNP or fragments thereof of at least 12 amino acids including BNP or NT-proBNP.

11. A method according to claims 1 to 10, wherein said level of HCT-proET-1 or fragments thereof of at least 12 amino acids is measured with a sandwich immunoassay.

## Patentansprüche

1. *In vitro* Verfahren zur Risikostratifizierung für das Auftreten maligner Tachyarrhythmien bei einem Patienten mit einer Herzerkrankung, welches Folgendes umfasst:
- Bestimmung des Spiegels von CT-proET-1 oder Fragmenten davon mit mindestens 12 Aminosäuren in einer einem Patienten entnommenen Probe.

2. *In vitro* Verfahren nach Anspruch 1, worin der Spiegel von CT-proET-1 oder Fragmenten davon mit mindestens 12 Aminosäuren mit dem Risiko maligner Tachyarrhythmien korreliert wird, die eine Implantation eines implantierbaren Kardioverter-Defibrillators (IDC) und/oder antiarrhythmische Hybridtherapie erfordert.

3. *In vitro* Verfahren nach Anspruch 2 zur Primärprävention von plötzlichem arrhythmiebedingtem Herztod.

4. Verfahren nach Anspruch 2 oder 3, wobei der korrelierende Schritt den Vergleich des Spiegels von CT-proET-1 oder Fragmenten davon mit mindestens 12 Aminosäuren mit einem Schwellenwert beinhaltet, wobei das Risiko einer Tachyarrhythmie für den Patienten sehr hoch ist, wenn der Spiegel von CT-proET-1 oder mindestens 12 Aminosäuren umfassenden Fragmenten davon oder Fragmente davon diesen Schwellenwert übersteigen.

5. Verfahren nach Anspruch 4, wobei der Schwellenwert bei etwa 73 +/- 20 % pmol/L liegt.

6. Verfahren nach Anspruch 1 bis 5, wobei die Herzerkrankung Herzinsuffizienz mit ischämischer oder nicht-ischämischer Ätiologie ist.

7. Verfahren nach Anspruch 1 bis 6, wobei die Patienten eine linksventrikuläre Ejektionsfraktion von ≤ 45 % aufweisen.

8. Verfahren nach einem der vorangehenden Ansprüche, wobei die Probe aus einer eine Blutprobe, eine Serumprobe und eine Plasmaprobe umfassenden Gruppe ausgewählt wird.

9. Verfahren nach einem der vorangehenden Ansprüche, das weiterhin das Verbinden des Spiegels von CT-proET-1 oder mindestens 12 Aminosäuren umfassenden Fragmenten davon mit dem Spiegel/Zustand eines oder mehrerer zusätzlicher Prognosemarker, Parameter oder Faktoren beinhaltet, wobei die Verbindung des Spiegels von CT-proET-1, oder mindestens 12 Aminosäuren umfassenden Fragmenten davon mit dem Spiegel/Zustand eines oder mehrerer zusätzlicher Prognosemarker, Parameter oder Faktoren den vorhersagbaren Risikowert dieses Verfahrens erhöht.

10. Verfahren nach Anspruch 9, wobei der zusätzliche Prognosemarker, Parameter oder Faktor aus einer Gruppe ausgewählt wird, die alle in Tabelle 2 gelisteten Parameter umfasst, die in der univariaten oder multivariaten Analyse p<0,05 aufweisen, wobei BNP nur ein Beispiel für proBNP oder mindestens 12 Aminosäuren umfassende Teile davon ist, die BNP oder NT-proBNP einschließen.

11. Verfahren nach den Ansprüchen 1 bis 10, wobei der Spiegel von CT-proET-1 oder mindestens 12 Aminosäuren umfassenden Fragmenten davon mit einem Sandwich-Immunoassay gemessen wird.

## Revendications

1. Procédé *in vitro* de stratification du risque de la survenue de tachyarythmies malignes pour un patient souffrant d'une cardiopathie comprenant :
la détermination du taux de CT-proET-1 ou de fragments de celle-ci d'au moins 12 acides aminés dans un échantillon obtenu d'un patient.

2. Procédé *in vitro* selon la revendication 1, dans lequel ledit taux de CT-proET-1 ou de fragments de celle-ci d'au moins 12 acides aminés est corrélé au risque de tachyarythmies malignes requérant l'implantation d'un défibrillateur automatique implantable (DAI), et/ou une thérapie hybride antiarythmique.

3. Procédé *in vitro* selon la revendication 2, pour la prévention primaire d'une mort subite arythmique.

4. Procédé selon la revendication 2 ou 3, dans lequel ladite étape de corrélation comprend la comparaison dudit taux de CT-proET-1 ou de fragments de celle-ci d'au moins 12 acides aminés à un taux seuil, de telle manière que lorsque ledit taux de CT-proET-1 ou de fragments de celle-ci d'au moins 12 acides aminés dépasse ledit taux seuil, ledit patient est à risque élevé de tachyarythmie.

5. Procédé selon la revendication 4, dans lequel ledit taux seuil est à environ 73 +/- 20 % pmol/l.

6. Procédé selon les revendications 1 à 5, dans lequel la cardiopathie est une insuffisance cardiaque d'étiologie soit ischémique soit non ischémique.

7. Procédé selon les revendications 1 à 6, dans lequel les patients présentent une fraction d'éjection VG ≤ 45 %.

8. Procédé selon l'une quelconque des revendications précédentes, dans lequel ledit échantillon est choisi dans le groupe comprenant un échantillon de sang, un échantillon de sérum et un échantillon de plasma.

9. Procédé selon l'une quelconque des revendications précédentes, comprenant en outre la combinaison dudit taux de CT-proET-1 ou de fragments de celle-ci d'au moins 12 acides aminés avec le taux/l'état d'un ou de plusieurs marqueurs, paramètres ou facteurs de pronostic supplémentaires, de telle manière que la combinaison dudit taux de CT-proET-1 ou de fragments de celle-ci d'au moins 12 acides aminés avec ledit taux/état de marqueur(s), paramètre (s) ou facteur(s) de pronostic supplémentaire (s) augmente la valeur prédictive du risque dudit procédé.

10. Procédé selon la revendication 9, dans lequel le marqueur, paramètre ou facteur de pronostic supplémentaire est choisi dans un groupe comprenant tous les paramètres énumérés dans le tableau 2, qui présentent une valeur p < 0,05 dans l'analyse à variable unique ou à variables multiples, de telle manière que le BNP est seulement un exemple pour le proBNP ou des fragments de celui-ci d'au moins 12 acides aminés y compris le BNP ou le NT-proBNP.

11. Procédé selon les revendications 1 à 10, dans lequel ledit taux de CT-proET-1 ou de fragments de celle-ci d'au moins 12 acides aminés est mesuré par un test immunologique de type sandwich.
